# EUROPÄISCHE PATENTSCHRIFT

(11) **EP 3 362 001 B1**
(45) Veröffentlichungstag und Bekanntmachung des Hinweises auf die Patenterteilung: **31.03.2021**
(21) Anmeldenummer: 16801706.9
(22) Anmeldetag: 11.11.2016
(51) Int. Cl.: A61F 2/52

(54) **BRUSTPROTHESE**
BREAST PROSTHESIS
PROTHÈSE MAMMAIRE

(30) Priorität: 13.11.2015 DE 202015007895 U
(43) Veröffentlichungstag der Anmeldung: 22.08.2018
(73) Patentinhaber: Amoena Medizin-Orthopädie-Technik GmbH, 83064 Raubling (DE)
(72) Erfinder: SELTER, Nils, 83112 Frasdorf (DE)
(74) Vertreter: Laufhütte, Dieter
(86) Internationale Anmeldenummer: PCT/EP2016/001880
(87) Internationale Veröffentlichungsnummer: WO 2017/080664

(56) Entgegenhaltungen:
- DE-B1- 2 457 041
- DE-U1- 29 516 281
- DE-U1-202004 011 988
- US-A- 6 042 608
- US-A1- 2011 245 921

## Beschreibung

Die Erfindung betrifft eine Brustprothese mit einem elastisch verformbaren Körper, dessen Außenoberfläche der Brustform nachgebildet ist.

Brustprothesen werden nach typischerweise krebsbedingten (Teil)Entfernungen der Brust aus kosmetischen Gründen eingesetzt. Neben einer der natürlichen Brust möglichst nahekommenden Form und Haptik soll insbesondere ein hoher Tragekomfort erreicht werden.

Im Stand der Technik ist es bekannt, die Innenseite der Prothese mit einer plastisch verformbaren Schichte zu versehen, die sich der Form des Narbengewebes anpasst. In diesem Zusammenhang sind beispielsweise die Druckschriften EP 0 768 068 A1, deren Familienmitglied DE29516281 U1 den Oberbegriff von Anspruch 1 offenbart,

EP 2 133 042 A1 oder WO 2014/078021 A1 zu nennen.

Bei diesen bekannten Prothesen kann jedoch das Problem auftreten, dass durch diese plastisch verformbare Schicht das Narbengewebe okklusiv abgeschlossen wurde. Dies kann zu Hitzestaus und Schweißbildung unter der Prothese führen, was dem Tragekomfort abträglich ist und auch zu Hautreaktionen führen kann.

Aufgabe der Erfindung ist es, eine Brustprothese bereitzustellen, deren Innenseite sich an die Form des Narbengewebes anpassen kann, ohne einen Luftaustausch zu verhindern.

Vor diesem Hintergrund betrifft die Erfindung eine Brustprothese nach Anspruch 1.

In einer Ausführungsform ist der äußere Körper schalenförmig: Der äußere Körper weist Der äußere Körper weist eine in einer äußeren Kammer eingeschlossene gummielastische Masse auf. Die äußere Kammer kann beispielsweise durch einen Folienbeutel gebildet werden. Bei der gummielastischen Masse kann es sich beispielsweise um eine Zweikomponenten-Silikonkautschuk-Masse handeln.

Der innere Körper weist Der innere Körper weist eine in einer inneren Kammer eingeschlossene plastisch verformbare Masse auf. Auch die innere Kammer kann beispielsweise durch einen Folienbeutel gebildet werden.

Bei der plastisch verformbaren Masse kann es sich um eine homogene oder heterogene Masse handeln. Die Masse kann thixotrope Eigenschaften aufweisen. Bei der Masse kann es sich um eine strukturviskose Masse mit Fließgrenze handeln. Beispiele umfassen geringvernetzte Silikongele, vorzugsweise solche mit wenigstens zwei Komponenten, welche gegebenenfalls ferner ein oder mehrere Additive enthalten können. Geeignete Additive umfassen Phasenwechselmaterialien, expandierte Leichtfüllstoffe, beispielsweise poröse oder gasgefüllte Partikel (im Rahmen des Herstellungsprozesses können unexpandierte Leichtfüllstoffe zugegeben werden, die durch Temperatur während des Herstellungsprozesses expandieren), Verdickungsmittel, Aerosile oder Thixotropierungsmittel. Beispiele weiterer geeigneter plastisch verformbarer Massen sind in der EP 0 768 068 A1, der EP 2 133 042 A1 und der WO 2014/078021 A1 offenbart.

Die innere Begrenzungsfolie des äußeren Körpers entpricht der äußeren Begrenzungsfolie des inneren Körpers und die äußere und innere Kammer werden durch drei entlang des gemeinsamen Umfangs verschweißte Kunststofffolien gebildet.

Die dreidimensionale Struktur bzw. das Textil ist vorzugsweise plastisch und/oder elastisch verformbar. Sie bzw. ist so geformt, dass eine Belüftung der Haut gewährleistet wird.

In einer Ausführungsform ist die dreidimensionale Struktur an die Innenoberfläche des inneren Körpers angeformt. In einer anderen Ausführungsform ist die dreidimensionale Struktur in die Innenoberfläche des inneren Körpers eingearbeitet. Beispielsweise kann vorgesehen sein, dass die dreidimensionale Struktur einen integralen Teil der Kunststofffolie bildet, welche die innere Begrenzung des inneren Körpers darstellt. Sie kann an dieser Folie angeformt bzw. in diese eingearbeitet sein.

In einer Ausführungsform ist die dreidimensionale Struktur auf die Innenoberfläche des inneren Körpers aufgeklebt oder aufgeschweißt.

In einer Ausführungsform handelt es sich bei der dreidimensionalen Struktur um einer Struktur aus Kunststoff. Bei dem Kunststoffmaterial kann es sich um dasselbe Material handeln, dass für die Kunststofffolie verwendet wird, welche die innere Begrenzung des inneren Körpers darstellt. Alternativ können für die Folie und die Struktur unterschiedliche Kunststoffmaterialien verwendet werden.

In einer Ausführungsform umfasst die dreidimensionale Struktur Kanäle, Stege und/oder Noppen.

In einer Ausführungsform ist das Textil auf die Innenoberfläche des inneren Körpers aufgeklebt oder aufgeschweißt.

In einer anderen Ausführungsform ist das Textil anhand eines Haltemittels an der Innenoberfläche des inneren Körpers angebracht. Beispielsweise kann vorgesehen sein, dass das Textil anhand einer auf die Innenoberfläche des inneren Körpers aufgeklebten oder aufgeschweißten Klettfläche an der Innenoberfläche des inneren Körpers angebracht ist.

In einer Ausführungsform handelt es sich bei dem Textil um ein dreidimensionales Textil mit einer Stärke von beispielsweise größer 2 mm und vorzugsweise von größer 5 mm. Beispiele umfassen zwei- oder mehrflächige Textile wie beispielsweise Abstandsgewirke oder Abstandsgestricke.

In einer Ausführungsform weist das Textil zumindest an der dem Körper zugewandten Oberfläche eine Oberflächenstruktur wie beispielsweise Rippen oder Noppen auf.

In einer Ausführungsform weist das Textil Naturfasern auf oder besteht daraus. Naturfasern können Vorteile in der Hautverträglichkeit und in einer schwachen Geruchsannahme haben. Beispiele geeigneter Naturfasern umfassen Baumwollfasern oder Wollfasern.

In einer Ausführungsform weist das Textil Kunstfasern auf oder besteht daraus. Kunstfasern können Vorteile in einer guten Schweißableitung und einer geringen Oberflächenrauheit haben.

Auch Kombinationen aus Natur- und Kunstfasern sind denkbar und von der Erfindung umfasst.

Weitere Einzelheiten und Vorteile ergeben sich aus den nachfolgend anhand der Figuren diskutierten Ausführungsbeispielen. In den Figuren zeigen:
- Figur 1:: eine perspektivische Ansicht einer erfindungsgemäßen Brustprothese;
- Figur 2:: eine Draufsicht auf die Innenoberfläche der in Figur 1 gezeigten Brustprothese; und
- Figur 3:: eine Draufsicht auf die Innenoberfläche einer weiteren Ausführungsform einer erfindungsgemäßen Brustprothese.

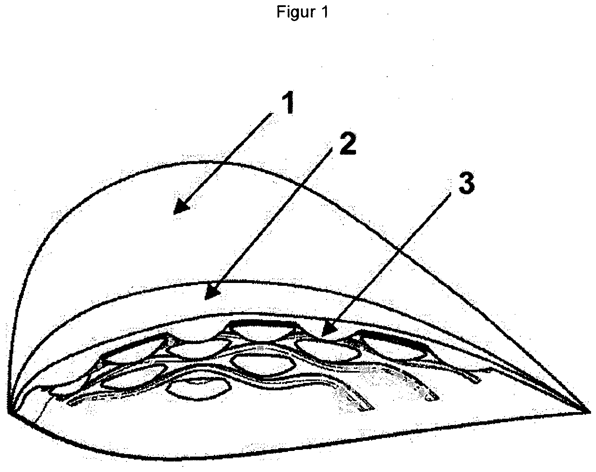

Figur 1 zeigt eine perspektivische Ansicht einer erfindungsgemäßen Brustprothese. Die Außenseite der Brustprothese wird durch einen schalenförmigen äußeren Körper 1 gebildet, welcher elastisch verformbar ist. An der Innenseite dieses schalenförmigen äußeren Körpers 1 ist ein ebenfalls schalenförmiger innerer Körper 2 angebracht.

Der äußere Körper 1 besteht aus zwei entlang dem Umfangsrand verschweißten Kunststofffolien, die eine Kammer bilden, in der sich eine gummielastische Zweikomponenten-Silikonkautschuk-Masse befindet.

Der innere Körper 2 wird durch Aufschweißen einer weiteren Folie an die Innenseite des äußeren Körpers 1 und das Füllen der zwischenliegenden Kammer mit einem Füllmaterial gebildet. Bei dem Füllmaterial handelt es sich um eine plastisch verformbare Masse mit thixotropen Materialeigenschaften, bei der es sich um ein gering vernetztes Silikongel zweier unterschiedlicher Silikonkomponenten handelt, das ferner Phasenwechselmaterial, expandierte Leichtfüllstoffe, Verdickungsmittel, Aerosole und Thixotropierungsmittel enthält.

Auf der Innenfolie des plastisch verformbaren Körpers 2 ist ein strukturiertes Kunststoff-Inlay 3 aufgeklebt. Die Klebeoberfläche des Inlays 3 ist glatt, um eine gute Haftung am Körper 2 zu gewährleisten.

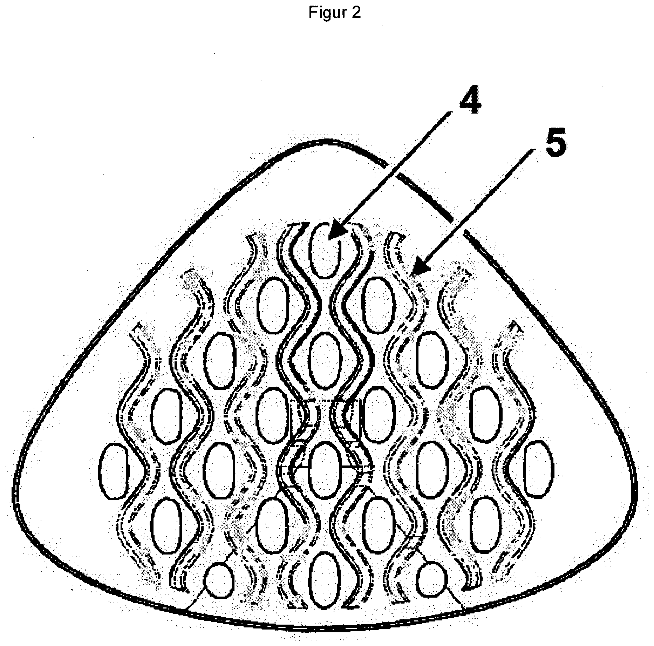

Die dem Körper zugewandte Oberfläche des Inlays 3 weist eine dreidimensionale Strukturierung auf, deren Gestalt der Figur 2 entnommen werden kann. Die Struktur umfasst eine regelmäßige Anordnung von Noppen 4 und Belüftungs- bzw. Faltkanälen 5. Die Noppen 4 sind in mehreren parallel verlaufenden Reihen angeordnet, wobei die Noppen in benachbarten Reihen um einen halben Noppenabstand versetzt sind. Alle Noppen 5 sind identisch ausgebildet und haben eine hügelig abgerundete Gestalt mit ovaler Grundfläche. Die Kanäle 5 verlaufen zickzackförmig zwischen den einzelnen Noppenreihen.

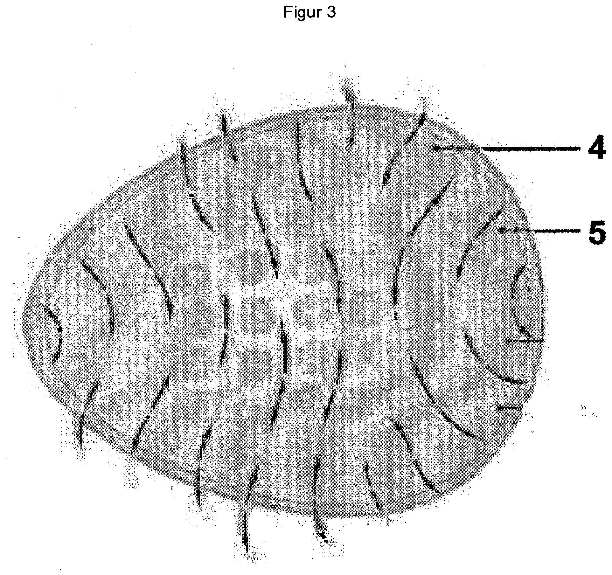

In Figur 3 wird ein weiteres Ausführungsbeispiel einer erfindungsgemäßen Brustprothese gezeigt, deren dreidimensionale Oberflächenstruktur sich von derjenigen des Ausführungsbeispiels gemäß Figuren 1 und 2 unterscheidet.

In der Ausführungsform gemäß Figur 3 sind die Noppen 5 abschnittsweise in gekrümmten Reihen und abschnittsweise gleichmäßig verteilt angeordnet. Sie haben eine hügelig abgerundete Gestalt mit kreisrunder Grundfläche. Die Größe der Noppen 5 nimmt vom Zentrum der Auflagefläche zum Rand der Auflagefläche hin ab. Zwischen den Noppen 5 sind Schlitze 4 einer definierten Länge in das Inlay 3 eingearbeitet, welche der Belüftung des Gewebes und der besseren Verformbarkeit des Inlays 3 dienen. Strömungswege der Luft durch die Struktur sind ist in Figur 3 anhand von Pfeilen angedeutet.

Durch das Vorsehen sowohl einer plastisch verformbaren Schichte 2 und einer dreidimensional geformten Belüftungsschicht 3 kann eine Anpassung an das unregelmäßige Narbengewebe erfolgen, ohne dass die Luftzufuhr an das Gewebe unterbrochen wird.

## Patentansprüche

1. Brustprothese mit einem elastisch verformbaren äußeren Körper (1), dessen Außenoberfläche der Brustform nachgebildet ist, und mit einem plastisch verformbaren inneren Körper (2), dessen Innenoberfläche sich an Unebenheiten des Gewebes anpassen kann, wobei der äußere Körper (1) eine in einer äußeren Kammer eingeschlossene gummielastische Masse aufweist und der innere Körper (2) eine in einer inneren Kammer eingeschlossene plastisch verformbare Masse aufweist, und wobei die äußere und innere Kammer durch drei entlang des gemeinsamen Umfangs verschweißte Kunststofffolien gebildet werden und die innere Begrenzungsfolie des äußeren Körpers (1) der äußeren Begrenzungsfolie des inneren Körpers (2) entspricht,
**dadurch gekennzeichnet,**
**dass** die Innenoberfläche des inneren Körpers (2) eine dreidimensionale Struktur (3) aufweist und/oder mit einem Textil (3) versehen ist, die bzw. das so ausgeführt ist, dass eine Belüftung der Gewebeoberfläche erreicht werden kann.

2. Brustprothese nach Anspruch 1, **dadurch gekennzeichnet, dass** der äußere Körper (1) schalenförmig ist und/oder dass es sich bei der in der äußeren Kammer eingeschlossenen gummielastischen Masse um eine Zweikomponenten-Silikonkautschuk-Masse handelt.

3. Brustprothese nach einem der vorhergehenden Ansprüche, **dadurch gekennzeichnet, dass** die dreidimensionale Struktur (3) an die Innenoberfläche des inneren Körpers (2) angeformt ist oder in diese eingearbeitet ist.

4. Brustprothese nach einem der vorhergehenden Ansprüche, **dadurch gekennzeichnet, dass** die dreidimensionale Struktur (3) auf die Innenoberfläche des inneren Körpers (2) aufgeklebt oder aufgeschweißt ist.

5. Brustprothese nach einem der vorhergehenden Ansprüche, **dadurch gekennzeichnet, dass** es sich bei der dreidimensionalen Struktur (3) um einer Struktur aus Kunststoff handelt.

6. Brustprothese nach einem der vorhergehenden Ansprüche, **dadurch gekennzeichnet, dass** die dreidimensionale Struktur (3) als Strukturelemente Kanäle (5), Stege und/oder Noppen (4) umfasst.

7. Brustprothese nach einem der vorhergehenden Ansprüche, **dadurch gekennzeichnet, dass** das Textil (3) auf die Innenoberfläche des inneren Körpers (2) aufgeklebt oder aufgeschweißt ist oder dass das Textil (3) anhand eines Haltemittels an der Innenoberfläche des inneren Körpers (2) angebracht ist, beispielsweise anhand einer auf die Innenoberfläche des inneren Körpers (2) aufgeklebten oder aufgeschweißten Klettfläche.

8. Brustprothese nach einem der vorhergehenden Ansprüche, **dadurch gekennzeichnet, dass** es sich bei dem Textil (3) um ein dreidimensionales Textil mit einer Stärke von beispielsweise größer 2 mm und vorzugsweise von größer 5 mm handelt, vorzugsweise um ein zwei- oder mehrflächiges Textil wie beispielsweise ein Abstandsgewirk oder ein Abstandsgestrick, und/oder dass das Textil (3) eine Oberflächenstruktur wie beispielsweise Rippen oder Noppen (4) aufweist.

9. Brustprothese nach einem der vorhergehenden Ansprüche, **dadurch gekennzeichnet, dass** das Textil (3) Naturfasern aufweist oder daraus besteht oder dass das Textil (3) Kunstfasern aufweist oder daraus besteht.

## Claims

1. A breast prosthesis having an elastically deformable outer body (1) whose outer surface is patterned on the shape of the breast and having a plastically deformable inner body (2) whose inner surface can adapt to irregularities of the tissue, wherein the outer body (1) has a rubber-elastic compound enclosed in an outer chamber and the inner body (2) has a plastically deformable compound enclosed in an inner chamber, and wherein the outer and inner chambers are formed by three plastic films welded along the total circumference and the inner boundary film of the outer body (1) corresponds to the outer boundary film of the inner body (2),
**characterized in that**
the inner surface of the inner body (2) has a three-dimensional structure (3) and/or is provided with a textile (3) that is designed such that a ventilation of the tissue surface can be achieved.

2. A breast prosthesis in accordance with claim 1, **characterized in that** the outer body (1) is of shell shape; and/or **in that** the rubber-elastic compound enclosed in the outer chamber is a two-component silicone rubber compound.

3. A breast prosthesis in accordance with one of the preceding claims, **characterized in that** the three-dimensional structure (3) is molded to the inner surface of the inner body (2) or is worked into it.

4. A breast prosthesis in accordance with one of the preceding claims, **characterized in that** the three-dimensional structure (3) is adhesively bonded or welded to the inner surface of the inner body (2).

5. A breast prosthesis in accordance with one of the preceding claims, **characterized in that** the three-dimensional structure (3) is a structure composed of plastic.

6. A breast prosthesis in accordance with one of the preceding claims, **characterized in that** the three-dimensional structure (3) comprises channels (5), webs and/or nubs (4) as structural elements.

7. A breast prosthesis in accordance with one of the preceding claims, **characterized in that** the textile (3) is adhesively bonded or welded to the inner surface of the inner body (2); or **in that** the textile (3) is attached to the inner surface of the inner body (2) using a holding means, for example using a hook and loop surface adhesively bonded or welded to the inner surface of the inner body (2).

8. A breast prosthesis in accordance with one of the preceding claims, **characterized in that** the textile (3) is a three-dimensional textile having a thickness of, for example, greater than 2 mm and preferably of greater than 5 mm, preferably a two-surface or multi-surface textile such as a spacer fabric or a knitted spacer fabric; and/or **in that** the textile (3) has a surface structure such as ribs or nubs (4).

9. A breast prosthesis in accordance with one of the preceding claims, **characterized in that** the textile (3) comprises or consists of natural fibers; or **in that** the textile (3) comprises or consists of plastic fibers.

## Revendications

1. Prothèse mammaire comprenant un corps extérieur (1) susceptible de subir une déformation élastique, dont la surface extérieure reproduit la forme du sein, et comprenant un corps intérieur (2), susceptible de subir une déformation plastique, dont la surface intérieure peut s'adapter à des irrégularités du tissu, le corps extérieur (1) comportant une masse élastique caoutchouteuse enfermée dans une chambre extérieure et le corps intérieur (2) comportant une masse susceptible de subir une déformation plastique enfermée dans une chambre intérieure, et la chambre extérieure et la chambre intérieure étant formées par trois films en matière plastique soudés le long de la périphérie commune et le film de délimitation intérieur du corps extérieur (1) correspondant au film de délimitation extérieur du corps intérieur (2),
**caractérisée en ce que**
la surface intérieure du corps intérieur (2) comporte une structure tridimensionnelle (3) et/ou est pourvue d'un textile (3), qui est réalisée ou réalisé de telle manière qu'une aération de la surface du tissu peut être obtenue.

2. Prothèse mammaire selon la revendication 1, **caractérisée en ce que** le corps extérieur (1) est en forme de coque et/ou **en ce que** la masse élastique caoutchouteuse enfermée dans la chambre extérieure est une masse à deux composants silicone/caoutchouc.

3. Prothèse mammaire selon l'une des revendications précédentes, **caractérisée en ce que** la structure tridimensionnelle (3) est formée sur la surface intérieure du corps intérieur (2) ou est incorporée dans celle-ci.

4. Prothèse mammaire selon l'une des revendications précédentes, **caractérisée en ce que** la structure tridimensionnelle (3) est collée ou soudée sur la surface intérieure du corps intérieur (2).

5. Prothèse mammaire selon l'une des revendications précédentes, **caractérisée en ce que** la structure tridimensionnelle (3) est une structure en matière plastique.

6. Prothèse mammaire selon l'une des revendications précédentes, **caractérisée en ce que** la structure tridimensionnelle (3) comprend des canaux (5), des nervures et/ou des protubérances (4) en guise d'éléments de structure.

7. Prothèse mammaire selon l'une des revendications précédentes, **caractérisée en ce que** le textile (3) est collé ou soudé sur la surface intérieure du corps intérieur (2) ou **en ce que** le textile (3) est appliqué sur la surface intérieure du corps intérieur (2) à l'aide d'un moyen de retenue, par exemple à l'aide d'une surface agrippante collée ou soudée sur la surface intérieure du corps intérieur (2).

8. Prothèse mammaire selon l'une des revendications précédentes, **caractérisée en ce que** le textile (3) est un textile tridimensionnel d'une épaisseur par exemple supérieure à 2 mm et de préférence supérieure à 5 mm, de préférence un textile à double surface ou à surfaces multiples, comme par exemple un tissu d'espacement tricoté en chaîne ou un tissu d'espacement tricoté en trame, et/ou **en ce que** le textile (3) comporte une structure de surface, comme par exemple des côtes ou des nopes (4).

9. Prothèse mammaire selon l'une des revendications précédentes, **caractérisée en ce que** le textile (3) comporte ou est constitué de fibres naturelles ou **en ce que** le textile (3) comporte ou est constitué de fibres synthétiques.
